# EUROPEAN PATENT SPECIFICATION

(11) **EP 0 591 378 B1**
(45) Date of publication and mention of the grant of the patent: **31.03.1999**
(21) Application number: 92914032.5
(22) Date of filing: 26.06.1992
(51) Int. Cl.: A61K 31/10, A61K 38/00, A61K 38/22, A61K 38/27, C07K 14/00, A61K 31/70, A61K 31/675

(54) **METHOD OF PREVENTING AND TREATING CHEMOTHERAPY-INDUCED ALOPECIA**
VERFAHREN ZUR VORBEUGUNG UND BEHANDLUNG VON DURCH CHEMOTHERAPIE AUSGELÖSTER ALOPEZIE
PROCEDE DE PREVENTION ET DE TRAITEMENT DE L'ALOPECIE INDUITE PAR CHIMIOTHERAPIE

(30) Priority: 28.06.1991 US 722500; 01.11.1991 US 786788; 20.12.1991 US 810412
(43) Date of publication of application: 13.04.1994
(73) Proprietor: Hairbiotech, Inc., Miami, Florida 33176 (US)
(72) Inventor: JIMENEZ, Joaquin J., Miami, FL 33144 (US); YUNIS, Adel A., Miami, FL 33183 (US)
(74) Representative: Zinnecker, Armin, Dipl.-Ing.
(86) International application number: US9205241
(87) International publication number: WO9300079

(56) References cited:
- US-A- 4 863 902
- US-A- 5 102 870
- J NATL CANCER INST,, VOL. 76, NO. 4, PAGE(S) 641-648, 1986 STUDZINSKI G P et al 'POTENTIATION BY 1-ALPHA 25 DIHYDROXYVITAMIN D-3 OF CYTOTOXICITY TO HL-60 CELLS PRODUCED BY CYTARABINE AND HYDROXYUREA'
- Annual Rev. Med., Volume 40, issued 1989, H. REICHEL et al., "Systemic effects of vitamin D", pages 71-78, Abstract.
- Arch. Dermatol. Res., Volume 279(4), issued 1987, K. KATSUOKA et al., "Effects of epidermal growth factor, fibroblast growth factor, minoxidil and hydrocortisone on growth kinetics in human hair bulb papilla cells and root sheath fibroblasts cultured in vitro", pages 247-250, Abstract.
- FASEB J., Volume 5, issued July 1991, J.J. JIMENEZ et al., "Interleukin-1 protects from cytosine arabinoside-induced alopecia in the rat model", pages 2456-2458, entire document.
- Science, Volume 249, issued 29 September 1990, A.M. HUSSEIN et al., "Protection from Chemotherapy-Induced alopecia in a rat model", pages 1564-1566, entire document.
- Cancer Research, Volume 38(4), issued 1990, J.J. JIMENEZ et al., "Stimulated monocyte-conditioned media protect from cytosine arabinoside-induced alopecia in rat", page 973A, entire document.
- Cancer Research, Volume 52, issued 15 January 1992, J.J. JIMENEZ et al., "Protection from 1-beta-D-arabinofuranosylcytosine-induced alopecia by epidermal growth factor and fibroblast growth factor in the rat model", pages 413-415, entire document.

## Description

### BACKGROUND

Alopecia is a common and distressing side effect of many chemotherapeutic agents and for which there is currently no effective preventive measure. In a recent study, thirty-five of forty-six patients receiving chemotherapy ranked alopecia as more important than vomiting (Tierney et al, *B. J. Cancer*, 62:527-528, 1990).

Recently, using the young rat model, Applicants demonstrated that ImuVert, a biologic response modifier prepared from the bacterium *Serratia marcescens*, protected the animals from alopecia induced by cytosine arabinoside or adriamycin (Hussein et al, *Science* 249: 1564-1566, 1990). In subsequent studies, similar protection from ARA-C-induced alopecia was observed from recombinant interleukin-1 (IL-1) beta (Jimenez et al *FASEB J*. 1991).

The present invention provides an independent method of preventing and treating chemotherapy-induced alopecia. This method involves the use of a growth factor, such as epidermal growth factor (EGF) or fibroblast growth factor (FGF). It should be noted that, as far as Applicants are aware, ImuVert has not been shown to stimulate the production of EGF or FGF, nor has it been proposed to stimulate such production.

The present invention also relates to the use of Vitamin D,, or a metabolite thereof, alone or in combination with EGF to prevent or treat alopecia. Vitamin D, is absorbed after ingestion of fish liver oils or irradiated yeast. Plants and animal sources contain only the inactive vitamin D precursors, 7-dehydrocholesterol or ergosterol. 7-Dehydrocholesterol is stored in the skin and can be converted by sunlight into vitamin D₃. However, whether ingested or formed by ultraviolet irradiation in the skin, Vitamin D has to be transformed into active metabolites. Vitamin D, is converted to 25-hydroxycholecalciferol by liver enzymes. Then in the kidneys two compounds 1,25-dihydroxycholecalciferol and 24,25-dihydroxycholecalciferol are formed. The vitamin D active metabolites play an important role in the absorption of calcium from the intestinal tract, bone deposition and bone reabsorption.

1,25-Dihydroxyvitamin D₃, an active metabolite of Vitamin D₃, has been shown to increase EGF receptors on breast cancer cells (Falette et al, Molec. and Cell. Endocrinol., 63 (1-2):189-198, 1989) and on a cell line established from rat calvaria (Petkovich et al, J. Biol. Chem. 262 (28):13424-13428, 1987). However, as far as Applicants are aware, the effect of vitamin D, or a metabolite thereof, on alopecia ham not been shown or proposed.

Document FASEB J., vol. 5, 1991, pages 2456 to 2458, describes a method of preventing or reducing chemotherapy-induced alopecia by administering IL-1.

Document Arch. Dermatol. Res., vol. 279 (4), 1987, pages 247 to 250, abstract shows that epidermal or fibroblast growth factor stimulate proliferation of human hair bulb papilla cells and hair root sheath fibroblasts in vitro.

It is an object of the invention to provide a medicament for treating or preventing or reducing alopecia in patients undergoing treatment with chemotherapeutic agents, including cycle specific agents (such as cytosine arabinoside (ARA-C)) and non cycle specific agents (such as Cytoxan), individually or in combination.

Further objects and advantages of the invention will be clear from the description that follows.

### BRIEF DESCRIPTION OF THE DRAWINGS

FIGURE 1 is a photograph of 10 rats from Experiment I, Table I (see below). All rats received ARA-C 50 mg/kg x 7 days. Five rats on top received buffer solution s.c. Five rats on bottom received murine EGF 2 µg s.c. daily x 7 days.
FIGURE 2 is a photograph of 6 rats from Experiment III, Table I (see below). All rats received ARA-C 50 mg/kg x 7 days. Three rats on top received buffer solution S.C. Three rats on bottom received rHu-EGF 2 µg S.c. daily x 7 days.
FIGURE 3 is a photograph of 4 rats from topical murine-EGF experiment (see below). All rats received ARA-C 50 mg/kg x 7 days. Two rats on the left received murine EGF 10 µg in DMSO daily x 7 days rubbed topically between the shoulder blades over an area of 1 cm². Two rats on the right received buffer solution topically.
FIGURE 4 is a photograph of 12 rats from ARA-C-aFGF experiment (see below). All rats received ARA-C 50 mg/kg x 7 days. Six rats on top received buffer solution s.c. Six rats on bottom received aFGF 2 µg s.c. daily x 7 days.
FIGURE 5 is a photograph of 8 rats treated with combination chemotherapy Cytoxan and Adriamycin. Four rats on top treated in addition with murine EGF. Four rats on bottom treated with buffer solution.
FIGURE 6 is a photograph of 10 rats treated with VP-16. All rats received 1.5 mg/kg x 3 days i.p. of VP-16. Five rats on top received buffer solution for four days prior to treatment. Five rats on bottom received Vitamin D, 50 µg/day for 4 days for four days prior to treatment.
FIGURE 7 is a photograph of 6 rats treated with combination chemotherapy Cytoxan and Adriamycin (25 mg/kg i.p. X 1 day and 2.5 mg/kg i.p. X 3 days, respectively). Three rats on top received buffer solution for four days prior to treatment. Three rats on bottom received Vitamin D, 50 µg/day for four days prior to treatment.
FIGURE 8(A-C). For each experiment, five day old rats were randomly divided into equal numbers. The experimental group of rats (top group) received 0.2 µg of 1,25-dihydroxyvitamin D₃ in 0.15 ml of absolute ethanol daily over the head and neck for 5 days. Control rats (bottom group) were similarly treated with 0.15 ml of absolute ethanol. One day after the last topical treatment, the rats from Fig. 8A were treated with Cytoxan (CTX), rats from Fig. 8B with the Etoposide (VP-16) regimen and rats from Fig. 8C with CTX + Adriamycin (ADM) regimen.
FIGURE 9. Twenty 5-day old rats were randomly divided into two groups of 10 rats each. The experimental group of rats (top group) received 0.1 µg of 1,25(OH)2D, in 0.1 ml of absolute ethanol daily over the head only for 5 days. Control rats (bottom group) were similarly treated with 0.1 ml of absolute ethanol. One day after the last topical treatment, all rats were treated with the VP-16 regimen.
FIGURE 10. Thirteen 9-day old rats were randomized into two groups. Experimental, 7 rats (top group), received 1 µg of RO 23-7553 in 0.2 ml absolute ethanol daily topically over the neck and back for 6 days. Control, 6 rats (bottom group), were similarly treated with 0.2 ml of absolute ethanol. One day after the last treatment all rats were treated with the VP-16 regimen.
FIGURE 11, shows the effect of 1,25-dihydroxyvitamin D₃ on hair growth.

### DETAILED DESCRIPTION OF THE INVENTION

The present invention relates generally to a method of preventing or reducing alopecia, particularly in patients undergoing chemotherapy. Applicants have shown that a growth factor, such as EGF, and Vitamin D₃ appear to render the hair follicle resistant to the toxic effect of chemotherapeutic agents thus preventing hair lose.

In one embodiment of the present method, a growth factor is administered to a patient undergoing chemotherapy in an amount sufficient to prevent or reduce the hair loss that normally accompanies this treatment regimen.

Growth factors suitable for use in the present method include EGF, FGF, transforming growth factors (TGF), and platelet-derived growth factor (PDGF). The growth factors can be derived from natural sources (for example, human tissue or rodent tissue); however, recombinant production is preferred as large quantities can be produced at relatively low cost. Chemically synthesized factors can also be used. The use of portions or derivatives of growth factors, such as EGF and FGF, is also contemplated as long as those portions or derivatives can effect the same result observed with the factor itself.

In another embodiment of the present invention, Vitamin D₃ or metabolite, analog, derivative or structural variant thereof (for example 1,25-dihydroxy-16-ene-23-yne-cholecalciferol; 1 α-hydroxyvitamin D₃; 1 α-24-dihydroxyvitamin D₃, MC 903, etc.) is administered to a warm blooded animal, for example, a human, in an amount sufficient to prevent or reduce the hair loss or stimulate hair growth. Hair loss treatable or preventable using vitamin D₃ can be due to chemotherapy or other cause, including, but not limited to, male pattern baldness. Examples of Vitamin D₃ metabolites suitable for use in the present method include, but are not limited to, 1,25-dihydroxyvitamin D₃ and 1,25-dihydroxy-16-ene-23-yne cholecalciferol.

Compositions suitable for use in the claimed method include as an active agent a growth factor, Vitamin D₃ (or a metabolite or analog thereof) or a combination of both. Such compositions can be formulated by combining an active agent together with a pharmaceutically acceptable vehicle (carrier, diluent or excipient), in an amount sufficient to effect the preventative effect when administered in accordance with an appropriately designed treatment protocol. The composition can be in dosage unit form.

Though not limiting the present method to a particular mode of action, it is suggested that Vitamin D₃ protects against alopecia by increasing the receptors for EGF at the hair follicle level. Accordingly, administering a combination of a growth factor and Vitamin D₃ can be expected to provide for greater protection.

Compositions suitable for use in the method to which the invention relates can be in a form suitable for topical administration. In that event, the composition can take the form of a solution, lotion, cream, gel or ointment. When the composition is to be administered by injection, it advantageously takes the form of a solution. The vehicle used, regardless of the form taken by the composition, can be inert or can itself possess a physiologically or pharmaceutically beneficial effect.

Various additives can be included in the composition. In this regard, inclusion in the composition of an agent that stimulates production of the patients' own growth factor is contemplated. Inclusion in compositions suitable for topical administration of penetration enhancing agents, such as DMSO or ethanol, is preferred. Stabilizers that extend shelf life can also be included in the composition, regardless of the manner in which it is formulated.

One skilled in the art will appreciate that various concentrations of growth factor and/or Vitamin D₃ can be used in the above-described composition. Optimum concentrations can be readily determined by one skilled in the art.

As noted above, the method to which the invention relates can involve either topical application of the active agent (a growth factor and/or Vitamin D₃ or metabolite thereof) or administration by injection. The amount of the active agent and the frequency of administration can vary depending on the individual and can readily be optimized by one skilled in the art. As an example, however, a solution of 2-100 µg/ml of 1,25-dihydroxyvitamin D₃ in absolute ethanol can be prepared and 3-5 ml of that solution applied directly to the scalp at various points with a dropper followed by scalp message for 3-5 min to ensure even distribution. When chemotherapy is involved, this treatment is, advantageously, administered once or twice daily beginning 5-8 days prior to initiation of chemotherapy and continued through the course of chemotherapy. However, it is also contemplated that the active agent can be administered substantially simultaneously with, or subsequent to, the administration of the chemotherapeutic agent.

The method to which the invention relates is shown in the Examples that follow to be effective when the cell cycle specific drug, ARA-C, is the chemotherapeutic agent used and when the combination of Adriamycin (cell cycle specific) and Cytoxan (non cell cycle specific) is used. It is contemplated, however, that, using the present method, hair loss resulting from treatment with other chemotherapeutic agents can be prevented. In addition, it is contemplated that a growth factor and/or vitamin D₃ can be used to prevent or retard hair loss in male pattern baldness if it is used on a regular basis and, advantageously, at the first sign of baldness, for example, once daily or every other day to the predisposed area of the scalp.

The alopecia preventative effect observed by Applicants was wholly unexpected Growth factors, such as EGF, are presumed stimulants of skin cell growth. Accordingly, these agents would be expected to induce the hair follicle to enter the cell cycle thus rendering the follicle more susceptible to chemotherapeutic agents, particularly cell cycle specific drugs, such as ARA-C. Thus, administration of growth factors to patients receiving chemotherapy would have been expected to aggravate hair loss. The reverse effect, however, was achieved. It should be noted therefore that the observations recorded herein with EGF and FGF are novel and have not been proposed or described in the literature. Similarly, nothing about the role of Vitamin D₃ in the body suggested that the vitamin would provide such excellent protection against alopecia, chemotherapeutically induced or otherwise.

The following non-limiting Examples describe certain aspects of the invention in greater detail.

### EXAMPLES

The following experimental details relate to Examples I-IV set forth below.

Sprague Dawley rats were purchased from Charles River Laboratories, Wilmington, MA. Cytosar-U (ARA-C) was from the Upjohn Company, Kalamazoo, MI. Receptor grade EGF from mouse submaxillary glands, human recombinant EGF, dimethyl sulfoxide (DMSO) and Vitamin D₃ were purchased from Sigma Chemical Co., St. Louis, MO. aFGF was purchased from AMGEN Corp., Thousand Oaks, CA.

All rats from each experiment were treated with ARA-C 50 mg/kg intraperitoneally (i.p.) daily for 7 days. For subcutaneous (s.c.) injections, EGF and FGF were prepared in PBS 1% BSA. Alopecia was always recorded on day 12 of experiment, and scored as previously described (Hussein et al, *Science*, 249:1564-1566, 1990; Jimenez et al, *FASEB J*., 1991).

For topical treatment, murine EGF was prepared as follows: One vial of EGF (100 µg) was dissolved in 0.2 ml of PBS 1% BSA and 0.12 ml of this solution was added to 0.48 of DMSO. Three hours prior to ARA-C injection, 0.1 ml of the EGF-DMSO mixture was applied to each rat over a 1 cm² area between the shoulders using a rubber tip applicator. Rats were then kept individually separated for a period of three hours, following which the treated area was carefully washed with soap and water and dried. Treatment was continued for 7 days. Control animals were similarly treated using DMSO without EGF.

### Example I

### Protective Effect of EGF

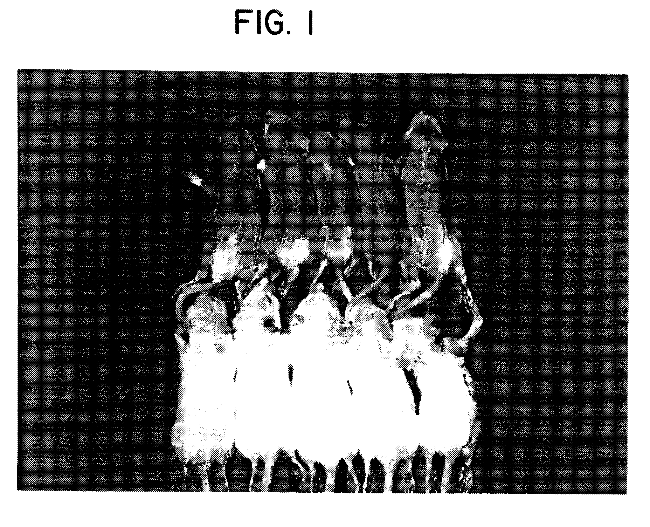

Two separate experiments were conducted to test the ability of murine EGF to protect from ARA-C-induced alopecia. In Experiment I, twenty-two 7-day old rats were randomized in two groups of eleven rats each. In addition to ARA-C, Group I received 2 µg of mouse EGF s.c. in the back between the two hind legs 3 hours prior to ARA-C injections daily for 7 days. Group II, received buffer solution similarly and served as control. Ten of eleven rats in Group II developed virtually total body alopecia and one rat developed more than 50% hair loss. In contrast, in Group I, 5 rats had no detectable hair loss and 6 rats had mild hair loss (Table I, Experiment I (FIGURE 1)). In Experiment II, twelve 7-day old rats were randomized in two groups of 6 rats each. In addition to ARA-C, Group I received mouse EGF 1 µg s.c. daily for 7 days. Group II received buffer s.c. All 6 rats in Group II developed moderately severe to severe alopecia, whereas in Group I, one rat had no detectable hair loss and 5 rats developed only minimal hair loss (Table I, Experiment II).

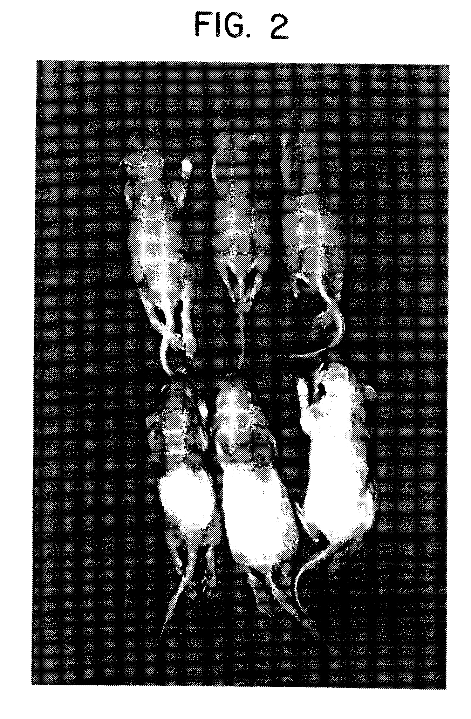

For the next experiment, rHu-EGF was used. Twelve 7-day old rats were randomized in two groups of six rats each. In addition to ARA-C, Group I received rHu-EGF 2 µg s.c. in the flank area daily for 7 days. Group II received buffer s.c. All 6 rats in Group II developed total body alopecia, whereas in Group I none of the rats had total body alopecia, one rat had no detectable hair loss, four rats had mild alopecia and one rat had moderate alopecia (Table I, Experiment III, (FIGURE 2)).

**TABLE I**

| OCCURRENCE OF ALOPECIA IN RATS TREATED WITH ARA-C. EFFECT OF MURINE EGF AND rHU-EGF. | | | | |
|---|---|---|---|---|
| | Alopecia* | | | |
| | 0 | 1+ | 2+ | 3+ |
| | Experiment I | | | |
| ARA-C | 0 | 0 | 1 | 10 |
| ARA-C + Murine EGF 2 µg | 5 | 6 | 0 | 0 |

| | Experiment II | | | |
|---|---|---|---|---|
| ARA-C | 0 | 0 | 3 | 3 |
| ARA-C + Murine EGF 1 µg | 1 | 5 | 0 | 0 |

| | Experiment III | | | |
|---|---|---|---|---|
| ARA-C | 0 | 0 | 0 | 6 |
| ARA-C + rHu-EGF 2 µg | 1 | 4 | 1 | 0 |
| Seven day old rats were used for all experiments. All rats received ARA-C 50 mg/kg x 7 days I.P. in 0.1 ml. Murine EGF and rHu-EGF in PBS 1% BSA were given 3 hours prior to ARA-C once daily in 0.1 ml s.c. x 7 days. Controls received PBS 1% BSA 0.1 ml s.c. x 7 days. Data recorded on day 12. No detectable alopecia 0; mild alopecia defined as less than 50% hair loss, 1+; moderately severe alopecia with more than 50% hair loss, 2+; and total or virtually total (90%) hair loss, 3+. | | | | |

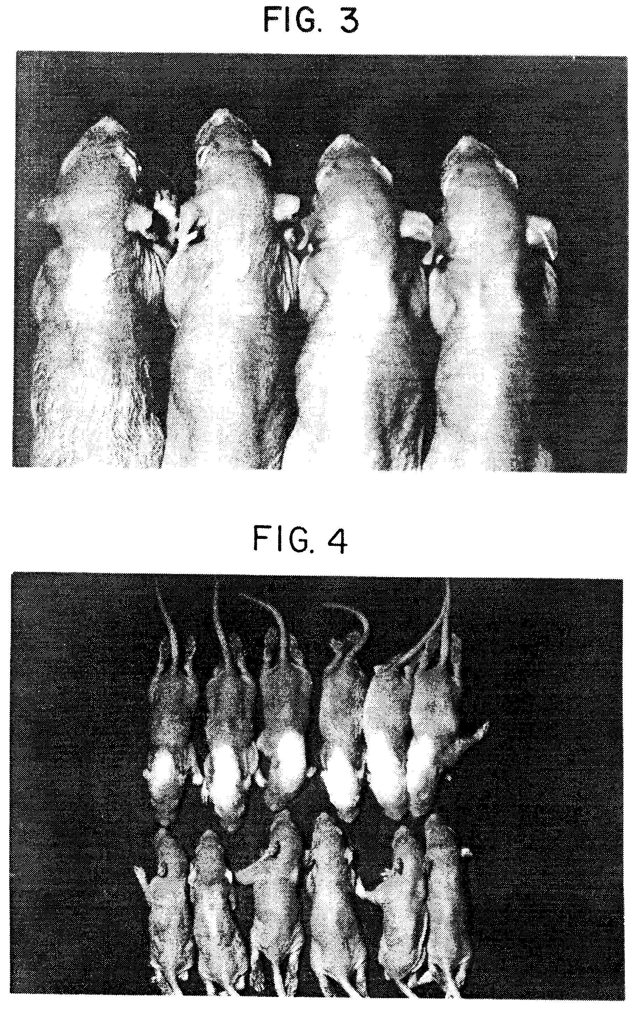

In the next experiment, twelve 7-day old rats were randomized in two groups of six rats each. Group I, in addition to ARA-C, received murine-EGF 10 µg in DMSO daily x 7 days rubbed topically with a cotton tip applicator between the shoulder blades over an area of 1 cm². Group II received control solution topically. In Group II, all six rats developed complete body alopecia. In Group I, all rats developed complete body alopecia except where the EGF was applied topically (FIGURE 3).

### Example II

### Protective Effect of aFGF

Fourteen 7-day old rats were randomized in two groups. All rats received ARA-C 50 mg/kg/day for seven days. In addition, Group I received aFGF 2µg s.c. on back of head daily for seven days. Group II received buffer injections and served as controls. Alopecia was recorded on day 12 of experiment. All rats in Group II developed complete body alopecia. In contrast, all rats in Group I were protected locally at the site of injection (FIGURE 4).

### Example III

### Protection from Cytoxan/Adriamycin-Induced Alopecia

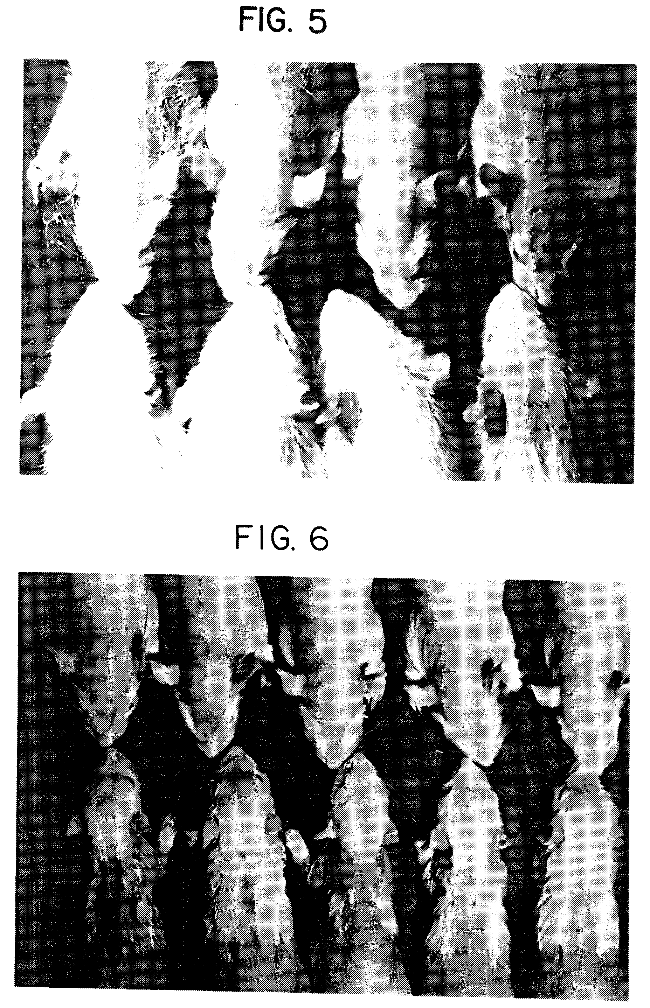

Eight 4-day old Sprague Dawley rats were randomized in two groups of 4 rats each. Group I, received EGF 2 µ s.c. on the head daily for 7 days. Group II received buffer injections and served as controls. One day after stopping EGF or buffer injections all rats received Cytoxan 25 mg/kg i.p. x 1 day and Adriamycin 2.5 mg/kg i.p. x 3 days. In Group II, all rats had 3+ alopecia over head and neck area. In contrast, in Group I one rat had mild alopecia, one rat minimal alopecia, and two rats no detectable alopecia over head and neck. (FIGURE 5). ImuVert when used under similar conditions did not protect from alopecia caused by the Cytoxan/Adriamycin combination.

### Example IV

### Protective Effect of Vitamin D₃

Twelve 7 day old rats were randomized in two groups. Group I was treated daily with buffer 0.1 ml s.c. for four days. The second Group was treated daily with Vitamin D₃ 50µg s.c. over head for four days. After stopping the buffer or Vitamin D₃ treatment, all rats received 1.5 mg/kg i.p. of VP-16 (Etoposide) daily for three days. All rats in Group I developed complete body alopecia while the rats in Group II were protected (FIGURE 6 shows 4 rats from each group).

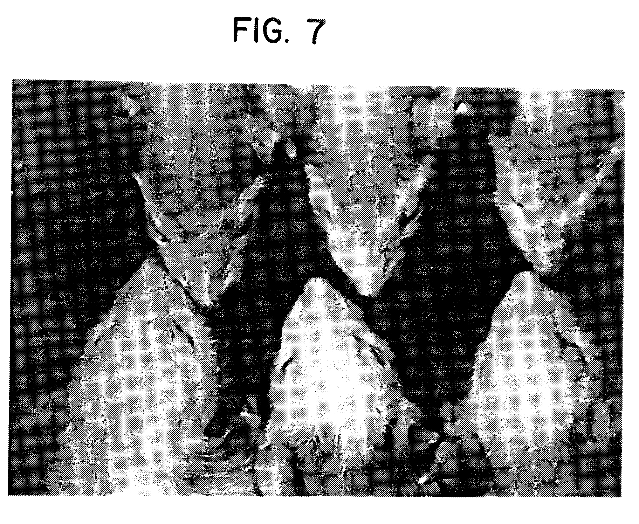

In other experiments, rats pretreated with Vitamin D₃ demonstrated excellent protection against alopecia produced by Etoposide, Cytoxan, Cytarabine and the combination of Cytoxan and Adriamycin (FIGURE 7). The results are set forth below in Table II.

**TABLE II**

| PROTECTION FROM CHEMOTHERAPY-INDUCED ALOPECIA BY PRETREATMENT WITH VITAMIN D₃. | | | |
|---|---|---|---|
| Alopecic drug tested | Total No. of experiments | Total No. of animals | Protection from alopecia |
| Etoposide (VP-16) | 2 | 22 | Yes* |
| Cytoxan (CTX) | 7 | 89 | Yes* |
| Cytarabine (ARA-C) | 1 | 8 | Yes* |
| Adriamycin + CTX Combination | 6 | 77 | Yes* |
| Chemotherapeutic agents were given as follows: VP-16 1.5 mg/kg i.p. daily for 3 days; Cytoxan 32.5 mg/kg as a single injection; ARA-C 50 mg/kg i.p. daily for 7 days; for combination (Adriamycin 2.5 mg/kg i.p. daily for 3 days plus Cytoxan 25 mg/kg as a single injection). Vitamin D₃ was given in 50 µg daily doses i.p.or s.c. for 4 days prior to chemotherapy. | | | |

| | | | |
|---|---|---|---|
| *In these experiments, protection from chemotherapy-induced alopecia was uniformly observed in all animals treated with Vitamin D₃ | | | |

In other experiments, 1,25-dihydroxyvitamin D₃ applied topically (0.5 µg daily) in 50% ethanol or DMSO also protected rats from VP-16-induced alopecia.

### EXAMPLE V

### Protective Effect of Vitamin D₃ Pretreatment

Topical Application of 1,25-dihydroxyvitamin D₃:
1,25-Dihydroxyvitamin D₃ was dissolved in absolute ethanol and applied topically with an applicator. Control animals were similarly treated with the same amount of ethanol. Animals were then kept individually separated for a period of three hours following which the treated area was carefully washed with soap and water and dried. Treatment was given daily beginning on day 5 after birth and ending on day 10.

Chemotherapy:
All chemotherapies were given I.P. and started at 11 days of age. CTX, 35 mg/kg, was given for one day only. VP-16, 1.5 mg/kg, was given for three days. For CTX and ADM combination, CTX, 25 mg/kg was given for one day and ADM, 2.5 mg/kg, for three days. At these doses neither CTX nor ADM alone will produce alopecia. Alopecia was recorded on the tenth day from beginning chemotherapy.

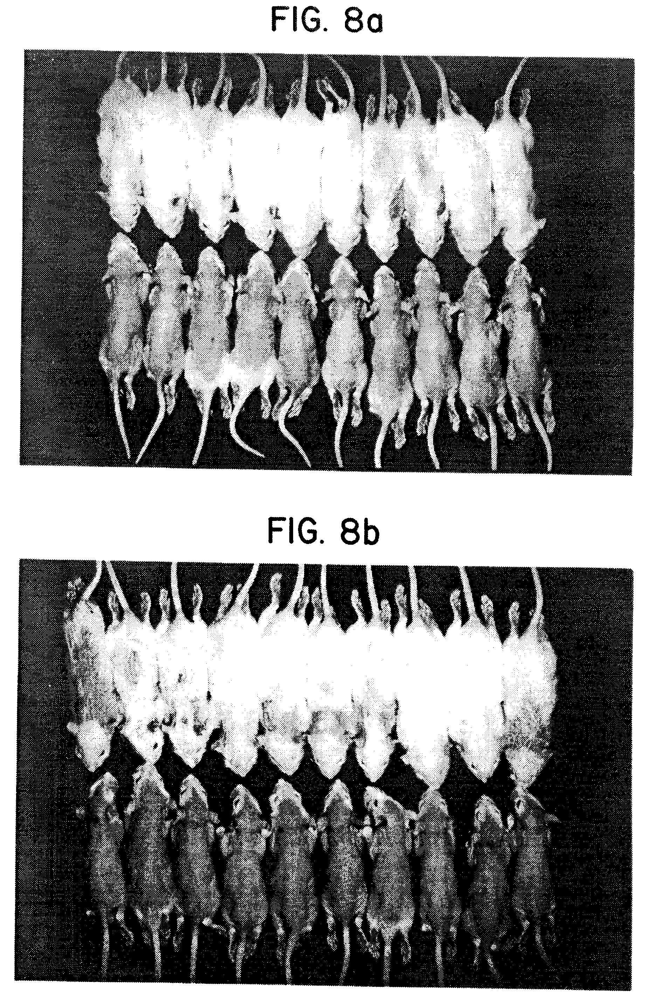

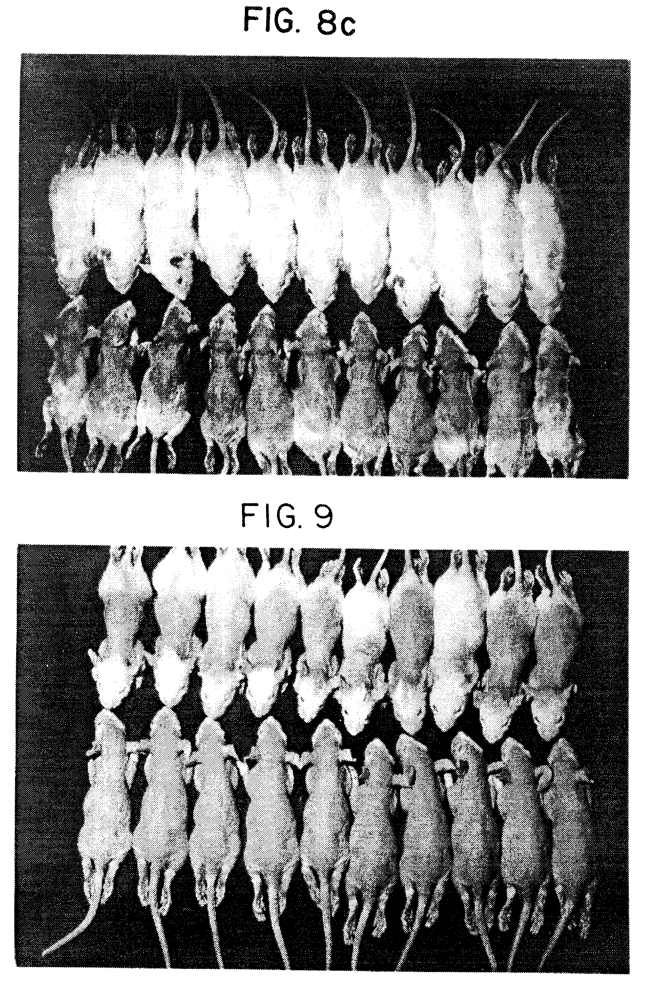

A total of 4 experiments were carried out. In the first experiment, protection from Cytoxan-induced alopecia was examined. The experimental group was pretreated with 0.2 µg of 1,25-dihydroxyvitamin D, in 0.15 ml of absolute ethanol applied topically over the head and neck and the control group received 0.15 ml of alcohol. All 10 rats in the control group became totally alopecic. In contrast, all animals in the experimental group were protected (Fig. 8A). The second experiment was carried out under similar conditions to examine protection from VP-16-induced alopecia. All 10 rats in the control group developed total body alopecia. In contrast, all rats in the experimental group were protected (Fig. 8B). The third experiment was designed to examine protection from alopecia induced by Cytoxan-Adriamycin combination. There were 11 rats in each group. Six rats in the control group developed alopecia over the head and neck and 5 rats developed total body alopecia. In contrast, all rats in the experimental group were protected (Fig. 8C). In the fourth experiment, protection from VP-16-induced alopecia was similarly examined except that the dose of 1,25-dihydroxyvitamin D₃ was reduced to 0.1 µg in 0.1 ml absolute ethanol applied topically over the head area only. All 10 rats in the control group became completely alopecic. In contrast, all rats in the experimental group were protected primarily at the site of 1,25-dihydroxyvitamin D₃ application (Fig. 9).

It is noteworthy that protection from 0.2 µg 1,25-dihydroxyvitamin D₃ was not limited to the site of application but involved the entire body, suggesting systemic absorption. When the dose was reduced to 0.1 µg applied to the head area only, protection from VP-16-induced alopecia was less generalized and was more limited to the site of application.

### EXAMPLE VI

### Protection from VP-16-Induced Alopecia by Topical Application of RO 23-7553

RO 23-7553 (1,25 dihydroxy-16-ene-23-yne-cholecalciferol) was dissolved in absolute ethanol and applied topically with an applicator. Control animals were similarly treated with the same amount of ethanol. Animals were then kept individually separated for a period of three hours following which the treated area was carefully washed with soap and water and dried. Treatment was given daily beginning on day 9 after birth and ending on day 14.

On day 15 all animals received VP-16, 1.5 mg/kg i.p., for three days. Alopecia was recorded on day 25.

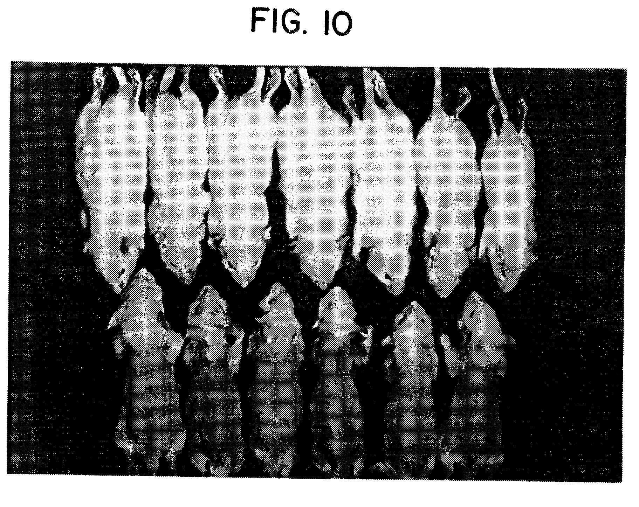

Thirteen rats were randomized in two groups. Experimental group, 7 rats; control group, 6 rats. The experimental group was pretreated with 1 µg of RO 23-7553 in 0.2 ml absolute ethanol applied topically over the neck and back and the control group received 0.2 ml of absolute ethanol. All six rats in the control group became totally alopecic over the neck and back. In contrast, all animals in the experimental group were protected (Fig. 10). It should be noted that when the chemotherapy is started at the age of 14 days, the head area does not become alopecic.

### Example VII

### Stimulation of Hair Growth by Vitamin D₃

During the course of the above-described studies on the protection from chemotherapy-induced alopecia by Vitamin D₃ and its active analog, 1,25-dihydroxyvitamin D₃, it was noted that rats treated with 1,25-dihydroxyvitamin D₃ not only were protected from chemotherapy-induced alopecia, but these rats had a better coat of hair and longer hair in the treated area. These observations prompted the following further experiments on the stimulation of hair growth by 1,25-dihydroxyvitamin D₃.

The backs of nineteen 25 day old Sprague Dawley rats were shaven and randomized in two groups.

Group I (control 10 rats) received 0.1 ml of ethanol applied topically once daily to the shaven area for 14 days.

Group II (Calcitriol 9 rats) received 50 ng of 1,25-dihydroxyvitamin D₃ in 0.1 ml of ethanol applied topically once daily to the shaven area for 14 days.

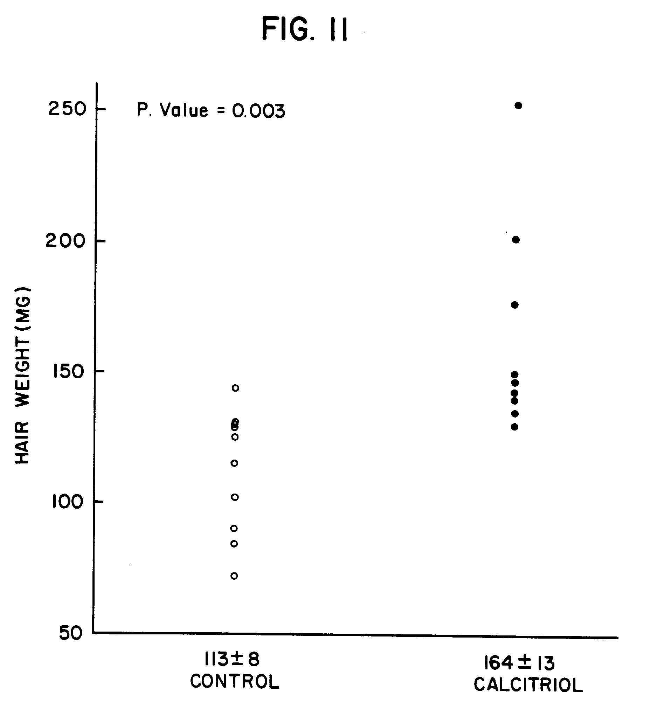

On day 15 stimulation of hair regrowth was assessed by reshaving an area 6 cm x 6 cm in diameter. The hair was collected and weighed. The difference between two groups was highly statistically significant. P. value 0.003 (see Table III and Figure 11).

**TABLE III**

| STIMULATION OF HAIR GROWTH BY CALCITRIOL IN RATS Hair Weight in Mg. | |
|---|---|
| Control | Calcitriol |
| 98 | 202 |
| 131 | 143 |
| 72 | 150 |
| 84 | 253 |
| 102 | 130 |
| 144 | 177 |
| 115 | 140 |
| 129 | 147 |
| 125 | 135 |
| 130 | |
| Mean S.E.M. | Mean S.E.M. |
| 113 ± 8 | 164 ± 13 |

Based on these data showing stimulation of hair growth by 1,23-dihydroxyvitamin D₃ administered topically in the rat, it is expected that 1,25-dihydroxyvitamin D₃ can be used as a stimulant of hair growth in cases of alopecia of any cause. Additionally, the data suggest that vitamin D, and its metabolites is/are necessary for optimal hair growth and therefore can be used to prevent hair loss from any cause, including male pattern baldness.

### Example VIII

### Formulations

The following are four formulations that include 1,25 -dihydroxyvitamin D₃ as active ingredient, and the methods of their manufacture.

### 1. TOPICAL SOLUTION

| Ingredients | % (W/W) |
|---|---|
| 1,25-Dihydroxyvitamin D₃ | 0.0002-0.10 |
| Propylene Glycol | 10.00 |
| Propylene Glycol Dicarprylate/Dicaprate^{a} | 30.00 |
| Butylated Hydroxytoluene (BHT) | 0.05 |
| Butylated Hydroxyanisole (BHA) | 0.05 |
| Ethyl Alcohol, Absolute q.s. to | 100.00 |

| | |
|---|---|
| ^{a} Can be substituted by the following materials (1) medium chain triglycerides; 2) dimethyl isosorbide; (3) polyethylene glycols; (4) ethoxydiglycol | |

### Manufacturing Procedure

i. Weigh the appropriate amount of propylene glycol dicaprylate/dicaprate, ethyl alcohol, propylene glycol in a stainless steel container.
ii.Dissolve BHT and BHA into the solution from step (i).
iii. Add the 1,25-dihydroxyvitamin D₃ into the mixture from step (ii) and stir until dissolved.

### 2. BUFFERED TOPICAL SOLUTION

| Ingredients | % (W/W) |
|---|---|
| 1,25-Dihydroxyvitamin D₃ | 0.0002-0.10 |
| Propylene Glycol | 50.00 |
| Hydroxypropyl cellulose (Klucel MF) | 0.50 |
| Methylparaben | 0.20 |
| Butylated Hydroxytoluene (BHT) | 0.05 |
| Butylated Hydroxyanisole (BHA) | 0.05 |
| Sodium Phosphate, Monobasic | 0.43 |
| Sodium Phosphate Dibasic | 0.70 |
| Sodium Hydroxide (q.s. to pH = 7) | 0.04 |
| Ethyl Alcohol, 95% Proof | 30.00 |
| Water q.s. to | 100.00 |

### Manufacturing Procedure

i. Dissolve the sodium phosphate, monobasic, sodium phosphate, dibasic, and sodium hydroxide in the water in a stainless steel container. Measure the pH of the solution. The pH of the solution should be 7.0; if not adjust the pH.
ii. Add the propylene glycol and ethyl alcohol to the solution from step (i).
iii. Dissolve the 1,25-dihydroxyvitamin D₃, methylparaben, BHT and BHA to the solution from step (ii).
iv. Dissolve Klucel MF to the solution from step (iii).

### 3. OIL-IN WATER BUFFERED TOPICAL LOTION

| Ingredients | % (W/W) |
|---|---|
| 1,25-dihydroxyvitamin D₃ | 0.0002-0.10 |
| Cetyl Alcohol | 0.25 |
| Stearyl Alcohol | 0.50 |
| Sorbitan Monosterate | 2.00 |
| Glyceryl Monostearate and Polyoxyethylene Stearate Blend (Arlacel 165) | 4.00 |
| Polysorbate 60 | 1.00 |
| Mineral Oil | 4.00 |
| Propylene Glycol | 5.00 |
| Butylated Hydroxyanisole | 0.05 |
| Propylparaben | 0.05 |
| Buffering Agent q.s. to pH | 7.00 |
| Sorbitol Solution | 2.00 |
| Edetate Disodium | 0.10 |
| Methylparaben | 0.18 |
| Water q.s. to | 100.00 |

### Manufacturing Procedure

i. Prepare the buffer solution (pH 7.0) in a stainless steel container.
ii. In a stainless steel vessel, at 70°C, melt the cetyl alcohol, stearyl alcohol, sorbitan monostearate, Arlacel 165, Polysorbate 60, mineral oil, butylated hydroxyanisole, propylparaben, and 50% propylene glycol together.
iii. Add the sorbitol solution to step (i) and heat the solution to 70°C.
iv. Add the edetate disodium and methylparaben to the solution from step (iii).
v. Dissolve the 1,25-dihydroxyvitamin D₃ in approximately 40% propylene glycol in a beaker and add this to the material from step (ii) while mixing. Rinse the container from 10% propylene glycol and add this to the mixture from step (ii).
vi. Add step (v) to step (iv) when both phases are at 70°C and homogenize. Cool the emulsion to 200m temperature.

### 4. TOPICAL GEL

| Ingredients | % (W/W) |
|---|---|
| 1,25-dihydroxyvitamin D₃ | 0.0002-0.10 |
| Butylated Hydroxytoluene (BHT) | 0.05 |
| Butylated Hydroxyanisole (BHA) | 0.05 |
| Hydroxypropyl Cellulose | 3.00 |
| Ethyl Alcohol, 95% Proof | 50.00 |
| Water q.s. to | 100.00 |

### Manufacturing Procedure

i. Weigh the ethyl alcohol and water in a stainless steel container.
ii. Dissolve the 1,25-dihydroxyvitamin D₃, BHT and BHA to the solution from step (i).
iii. Dissolve the hydroxypropyl cellulose to the solution from step (ii).

The entire contents of all references cited above are incorporated herein by reference.

While the present invention has been described in some detail for purposes of clarity and understanding, one skilled in the art will appreciate that various changes in form and detail can be made without departing from the true scope of the invention.

## Claims

1. Use of vitamin D₃, or derivative, analog or structural analog thereof, for the manufacture of a medicament to be administered to a patient undergoing chemotherapy for preventing or reducing chemotherapy-induced alopecia.

2. Use according to claim 1, characterized in that said derivative is 1,25-dihydroxyvitamin D₃, or 1,25-dihydroxy-16-ene-23-yne-cholecalciferol.

3. Use according to claim 1 or 2, characterized in that said vitamin D₃ or derivative, analog or strucural analog thereof is used for the manufacture of a medicament to be administered topically.

4. Use according to any one of claims 1-3, characterized in that said vitamin D₃ or derivative, analog or structural analog thereof is used for the manufacture of a medicament to be administered to a patient undergoing chemotherapy by a chemotherapeutic agent which is cell cycle specific.

5. Use according to claim 4, characterized in that said chemotherapeutic agent is cytosine arabinoside.

6. Use according to any one of claims 1-3, characterized in that said vitamin D₃ or derivative, analog or structural analog thereof is used for the manufacture of a medicament to be administered to a patient undergoing chemotherapy by a chemotherapeutic agent which is non cell cycle specific.

7. Use according to claim 6, characterized in that said chemotherapeutic agent is Cytoxan.

8. Use according to any one of claims 1-3, characterized in that said vitamin D₃ or derative, analog or structural analog thereof is used for the manufacture of a medicament to be administered to a patient undergoing chemotherapy by a chemotherapeutic agent which is a cell cycle specific agent in combination with a non cell cycle specific agent.

9. Use according to any one of claims 1-8, characterized in that said vitamin D₃ or derivative, analog or structural analog thereof is used for the manufacture of a medicament to be administered to a patient undergoing chemotherapy prior to the initiation of said chemotherapy.

10. Use according to claims 1-9, characterized in that further at least one proteinaceous growth factor is used for the manufacture of a medicament to be administered to a patient undergoing chemotherapy for preventing or reducing chemotherapy-induced alopecia.

11. Use according to claim 10, characterized in that said growth factor is epidermal growth factor.

12. Use according to claim 10, characterized in that said growth factor is fibroblast growth factor.

13. Use of vitamin D₃ or metabolite, analog or derivative thereof for the manufacture of a medicament to be administered to a warm-blooded animal for stimulating hair growth.

14. Use according to claim 13, characterized in that further a proteinaceous growth factor is used for the manufacture of a medicament to be administered to a warm-blooded animal for stimulating hair growth.

15. Use according to claim 14, characterized in that said growth factor is epidermal growth factor.

16. Use according to any one of claims 13-15, characterized in that said vitamin D₃ or metabolite, analog or derivative thereof is used for the manufacture of a medicament to be administered to a human suffering from male pattern baldness.

17. Use of a composition comprising an effective amount of a combination of vitamin D₃, or metabolite, analog or derivative thereof, and a proteinaceous growth factor, together with a pharmaceutically acceptable carrier, for the manufacture of a medicament for stimulating hair growth.

18. Use according to claim 17, characterized in that said composition is in the form of a gel, cream, lotion or ointment.

## Patentansprüche

1. Verwendung von Vitamin D₃, oder einem Derivat, Analogon oder strukturellen Analogon davon, zur Herstellung eines Medikamentes, das einem einer Chemotherapie unterzogenen Patienten zur Verhinderung oder Verminderung von chemotherapeutisch induzierter Alopecia verabreicht wird.

2. Verwendung gemäß Anspruch 1, dadurch gekennzeichnet, daß das Derivat 1,25-Dihydroxyvitamin D₃ oder 1,25-Dihydroxy-16-en-23-in-cholecalciferol ist.

3. Verwendung gemäß Anspruch 1 oder 2, dadurch gekennzeichnet, daß das Vitamin D₃ oder Derivat, Analogon oder strukturelle Analogon davon zur Herstellung eines Medikamentes verwendet wird, welches topisch verabreicht wird.

4. Verwendung gemäß mindestens einem der Ansprüche 1 - 3, dadurch gekennzeichnet, daß das Vitamin D₃ oder Derivat, Analogon oder strukturelle Analogon davon zur Herstellung eines Medikamentes verwendet wird, welches einem Patienten zu verabreichen ist, der einer Chemotherapie mit einem chemotherapeutischen Mittel, das Zellcyclus-spezifisch ist, unterzogen wird.

5. Verwendung gemäß Anspruch 4, dadurch gekennzeichnet, daß das chemotherapeutische Mittel Cytosinarabinosid ist.

6. Verwendung gemäß mindestens einem der Ansprüche 1 - 3, dadurch gekennzeichnet, daß das Vitamin D₃ oder Derivat, Analogon oder strukturelle Analogon davon zur Herstellung eines Medikamentes verwendet wird, welches einem Patienten zu verabreichen ist, der einer Chemotherapie mit einem chemotherapeutischen Mittel, das nicht Zellcyclus-spezifisch ist, unterzogen wird.

7. Verwendung gemäß Anspruch 6, dadurch gekennzeichnet, daß das chemotherapeutische Mittel Cytoxan ist.

8. Verwendung gemäß mindestens einem der Ansprüche 1 - 3, dadurch gekennzeichnet, daß das Vitamin D₃ oder Derivat, Analogon oder strukturelle Analogon davon zur Herstellung eines Medikamentes verwendet wird, welches einem Patienten zu verabreichen ist, der einer Chemotherapie mit einem chemotherapeutischen Mittel, das ein Zellcyclusspezifisches Mittel ist, in Kombination mit einem nicht Zellcyclus-spezifischem Mittel unterzogen wird.

9. Verwendung gemäß mindestens einem der Ansprüche 1 - 8, dadurch gekennzeichnet, daß das Vitamin D₃ oder Derivat, Analogon oder strukturelle Analogon davon zur Herstellung eines Medikamentes verwendet wird, welches einem eine Chemotherapie zu unterziehenden Patienten zu verabreichen ist, bevor die Chemotherapie begonnen wird.

10. Verwendung gemäß den Ansprüchen 1 - 9, dadurch gekennzeichnet, daß ferner mindestens ein proteinartiger Wachstumsfaktor zur Herstellung eines Medikamentes verwendet wird, das einem einer Chemotherapie unterzogenen Patienten zur Verhinderung oder Verminderung von chemotherapeutisch induzierter Alopecia verabreicht wird.

11. Verwendung gemäß Anspruch 10, dadurch gekennzeichnet, daß der Wachstumsfaktor epidermaler Wachstumsfaktor ist.

12. Verwendung gemäß Anspruch 10, dadurch gekennzeichnet, daß der Wachstumsfaktor Fibroblasten-Wachstumsfaktor ist.

13. Verwendung von Vitamin D₃ oder Metabolit, Analogon oder Derivat davon zur Herstellung eines Medikamentes, das einem warmblütigen Tier zur Stimulation des Haarwachstums verabreicht wird.

14. Verwendung gemäß Anspruch 13, dadurch gekennzeichnet, daß ferner mindestens ein proteinartiger Wachstumsfaktor zur Herstellung eines Medikamentes verwendet wird, das einem warmblütigen Tier zur Stimulation des Haarwachstums verabreicht wird.

15. Verwendung gemäß Anspruch 14, dadurch gekennzeichnet, daß der Wachstumsfaktor epidermaler Wachstumstaktor ist.

16. Verwendung gemäß mindestens einem der Ansprüche 13 - 15, dadurch gekennzeichnet, daß das Vitamin D₃ oder Metabolit, Analogon oder Derivat davon zur Herstellung eines Medikamentes verwendet wird, das einem Menschen, der an männlicher Glatzenbildung leidet, zu verabreichen ist.

17. Verwendung einer Zusammensetzung, umfassend eine wirksame Menge einer Kombination von Vitamin D₃ oder Metabolit, Analogon oder Derivat davon und einem proteinartigen Wachstumsfaktor, zusammen mit einem pharmazeutisch annehmbaren Träger, zur Herstellung eines Medikamentes zur Stimulation des Haarwachstums.

18. Verwendung gemäß Anspruch 17, dadurch gekennzeichnet, daß die Zusammensetzung in Form eines Gels, einer Creme, einer Lotion oder einer Salbe vorliegt.

## Revendications

1. Utilisation de vitamine D₃ ou d'un dérivé, d'un analogue ou d'un analogue structurel de celle-ci, pour la fabrication d'un médicament destiné à être administré à un patient subissant une chimiothérapie pour empêcher ou réduire l'alopécie induite par chimiothérapie.

2. Utilisation selon la revendication 1, caractérisée en ce que ledit dérivé est de la 1,25-dihydroxyvitamine D₃, ou du 1,25-dihydroxy-16-ène-23-yne-cholécalciférol.

3. Utilisation selon les revendications 1 ou 2, caractérisée en ce que ladite vitamine D₃ ou le dérivé, l'analogue ou l'analogue structurel de celle-ci est utilisé pour la fabrication d'un médicament destiné à être administré de manière topique.

4. Utilisation selon l'une quelconque des revendications 1 à 3, caractérisée en ce que ladite vitamine D₃ ou le dérivé, l'analogue ou l'analogue structurel de celle-ci est utilisé pour la fabrication d'un médicament destiné à être administré à un patient subissant une chimiothérapie par un agent chimiothérapeutique qui est spécifique quant au cycle cellulaire.

5. Utilisation selon la revendication 4, caractérisée en ce que ledit agent chimiothérapeutique est de la cytosine arabinoside.

6. Utilisation selon l'une quelconque des revendications 1 à 3, caractérisée en ce que ladite vitamine D₃ ou le dérivé, l'analogue ou l'analogue structurel de celle-ci est utilisé pour la fabrication d'un médicament destiné à être administré à un patient subissant une chimiothérapie par un agent chimiothérapeutique qui n'est pas spécifique quant au cycle cellulaire.

7. Utilisation selon la revendication 6, caractérisée en ce que ledit agent chimiothérapeutique est du Cytoxan.

8. Utilisation selon l'une quelconque des revendications 1 à 3, caractérisée en ce que ladite vitamine D₃ ou le dérivé, l'analogue ou l'analogue structurel de celle-ci est utilisé pour la fabrication d'un médicament destiné à être administré à un patient subissant une chimiothérapie par un agent chimiothérapeutique qui est un agent spécifique quant au cycle cellulaire en combinaison avec un agent non spécifique quant au cycle cellulaire.

9. Utilisation selon l'une quelconque des revendications 1 à 8, caractérisée en ce que ladite vitamine D₃ ou le dérivé, l'analogue ou l'analogue structurel de celle-ci est utilisé pour la fabrication d'un médicament destiné à être administré à un patient subissant une chimiothérapie avant le début de ladite chimiothérapie.

10. Utilisation selon les revendications 1 à 9, caractérisée en ce qu'un autre facteur de croissance protéinacé au moins est utilisé pour la fabrication d'un médicament destiné à être administré à un patient subissant une chimiothérapie pour empêcher ou réduire l'alopécie induite par chimiothérapie.

11. Utilisation selon la revendication 10, caractérisée en ce que ledit facteur de croissance est un facteur de croissance épidermique.

12. Utilisation selon la revendication 10, caractérisée en ce que ledit facteur de croissance est un facteur de croissance fibroblaste.

13. Utilisation de la vitamine D₃ ou d'un métabolite, d'un analogue ou d'un dérivé de celle-ci pour la fabrication d'un médicament destiné à être administré à un animal de sang chaud pour stimuler la croissance pileuse.

14. Utilisation selon la revendication 13, caractérisée en ce qu'un facteur de croissance protéinacé supplémentaire est utilisé pour la fabrication d'un médicament destiné à être administré à un animal de sang chaud pour stimuler la croissance pileuse.

15. Utilisation selon la revendication 14, caractérisée en ce que ledit facteur de croissance est un facteur de croissance épidermique.

16. Utilisation selon l'une quelconque des revendications 13 à 15, caractérisée en ce que ladite vitamine D₃ ou le métabolite, l'analogue ou le dérivé de celle-ci est utilisé pour la fabrication d'un médicament destiné à être administré à un être humain souffrant d'un calvitie de type mâle.

17. Utilisation d'une composition comportant une quantité efficace d'une combinaison de vitamine D₃, ou d'un métabolite, d'un analogue ou d'un dérivé de celle-ci, et d'un facteur de croissance protéinacé, ensemble avec un support pharmaceutiquement acceptable pour la fabrication d'un médicament destiné à stimuler la croissance pileuse.

18. Utilisation selon la revendication 17, caractérisé en ce que ladite composition se présente sous forme d'un gel, d'une crème, d'une lotion ou d'un onguent.
